# EUROPEAN PATENT APPLICATION

(11) **EP 3 950 319 A1**
(43) Date of publication of application: **09.02.2022**
(21) Application number: 20778329.1
(22) Date of filing: 10.03.2020
(51) Int. Cl.: B32B 15/08, A61B 5/0408, B32B 7/02, H05K 1/03

(54) **ELECTRODE JOINING STRUCTURE AND BIOLOGICAL SENSOR**

(30) Priority: 26.03.2019 JP 2019058327
(71) Applicant: Nitto Denko Corporation, Ibaraki-shi, Osaka 567-8680 (JP)
(72) Inventor: YOSHIOKA, Ryoma, Ibaraki-shi, Osaka 567-8680 (JP); MINAKATA, Masayuki, Ibaraki-shi, Osaka 567-8680 (JP)
(74) Representative: Hoffmann Eitle
(86) International application number: PCT/JP2020/010312
(87) International publication number: WO 2020/195796

(57) **Abstract**

An electrode joining structure and a biosensor in which breaking of wires is suppressed, are provided.

A biosensor includes a pressure-sensitive adhesive layer having an affixing surface to be affixed to a test subject; an electrode containing a conductive polymer having elasticity, and configured to be exposed from the affixing surface of the pressure-sensitive adhesive layer; a base material layer provided to be overlaid on a surface opposite to the affixing surface of the pressure-sensitive adhesive layer, and having tackiness represented by a maximum diameter from among diameters of steel balls that stop in a ball rolling method called J. Dow ball tack, the maximum diameter being greater than or equal to 0.4 mm and less than or equal to 4 mm; a substrate provided on the base material layer; a wire provided on the substrate and connected to the electrode; and a joining part joining the electrode and the wire.

## Description

### TECHNICAL FIELD

The present invention relates to an electrode joining structure and a biosensor.

### BACKGROUND ART

Conventionally, a biosensor that uses a biocompatible polymer substrate including a plate-like first polymer layer; a plate-like second polymer layer; electrodes; and a module for obtaining data, is known (see, for example, Patent Document 1).

### RELATED ART DOCUMENTS

### [Patent Document]

Patent Document 1: Japanese Laid-Open Patent Application No. 2012-010978

### SUMMARY OF THE INVENTION

### [PROBLEM TO BE SOLVED BY THE INVENTION]

A type of biosensor that can be affixed to the skin of a living body as the test subject, has elasticity to a certain extent, in order to be capable of following the motion of the living body. Such a biosensor may be partially stretched while following the motion of the living body. Also, such stretching may also occur when the biosensor is being affixed to the living body, or when the biosensor is being peeled off from the living body, and the like.

Meanwhile, a biosensor has wires that connect the electrodes to a control unit such as the module for obtaining data. If the biosensor is stretched, the wires may break.

Thereupon, the present invention has an object to provide an electrode joining structure and a biosensor in which breaking of the wires can be suppressed.

### [MEANS FOR SOLVING THE PROBLEM]

A biosensor according to an embodiment of the present invention includes a pressure-sensitive adhesive layer having an affixing surface to be affixed to a test subject; an electrode containing a conductive polymer having elasticity, and configured to be exposed from the affixing surface of the pressure-sensitive adhesive layer; a base material layer provided to be overlaid on a surface opposite to the affixing surface of the pressure-sensitive adhesive layer, and having tackiness represented by a maximum diameter from among diameters of steel balls that stop in a ball rolling method called J. Dow ball tack, the maximum diameter being greater than or equal to 0.4 mm and less than or equal to 4 mm; a substrate provided on the base material layer; a wire provided on the substrate and connected to the electrode; and a joining part joining the electrode and the wire.

### [ADVANTAGEOUS EFFECT OF THE PRESENT INVENTION]

An electrode joining structure and a biosensor in which breaking of the wires is suppressed, can be provided.

### [BRIEF DESCRIPTION OF THE DRAWINGS]

FIG. 1 is an exploded view illustrating a biosensor 100 according to an embodiment;
FIG. 2 is a diagram illustrating a cross section in a completed state corresponding to a cross section viewed in the direction of arrows A-A in FIG. 1;
FIG. 3 is a diagram illustrating a circuit configuration of the biosensor 100;
FIG. 4 is a diagram illustrating a ball rolling device 500;
FIG. 5 is a table showing test results; and
FIG. 6 is an exploded view illustrating a biosensor 100M4 according to a modified example.

### EMBODIMENTS FOR CARRYING OUT THE INVENTION

In the following, an electrode joining structure and a biosensor according to embodiments of the present invention will be described.

### <Embodiments>

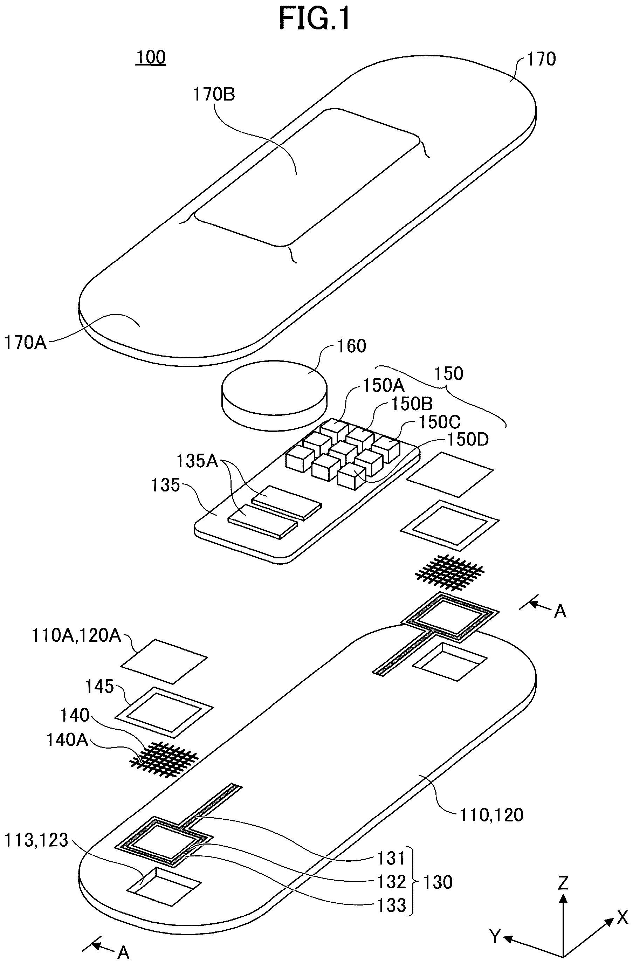

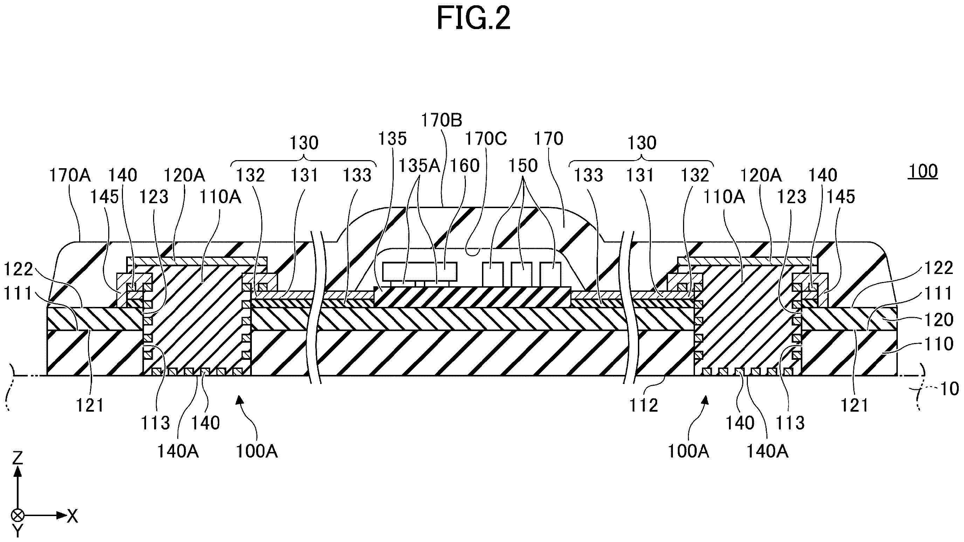

FIG. 1 is an exploded view illustrating a biosensor 100 according to an embodiment. FIG. 2 is a diagram illustrating a cross section in a completed state corresponding to a cross section viewed in the direction of arrows A-A in FIG. 1. The biosensor 100 according to the embodiment includes a pressure-sensitive adhesive layer 110, a base material layer 120, circuit parts 130, a substrate 135, probes 140, fixing tapes 145, an electronic device 150, a battery 160, and a cover 170, as major components. Among these elements, the pressure-sensitive adhesive layer 110, the base material layer 120, the circuit parts 130, and the probes 140 constitute an electrode joining structure 100A.

In the following, an XYZ coordinate system is defined for the description. Also, in the following, for the sake of convenience of description, the +Z axis direction side will be referred to as the upper side or the top, and the -Z axis direction side will be referred to as the lower side or the bottom; however, these do not represent a universal vertical relationship.

In the present embodiment, as an example, the biosensor 100 that is affixed to a living body as the test subject to measure biological information, will be described. The living body here includes a human body (a person) and living bodies other than a human body, and the biosensor is affixed to the skin, scalp, forehead, or the like of these bodies. In the following, the respective members constituting the biosensor 100 will be described.

In the following, each electrode contacting a living body as the test subject will be referred to as the probe 140, and will be described with the fixing tape 145 as an example of a joining part.

The biosensor 100 is a sheet-like member having a generally elliptic shape in plan view. The biosensor 100 is covered with the cover 170 on the top surface opposite to the bottom surface (a surface on the -Z direction side) that is to be affixed to a skin 10 of a living body. The bottom surface of the biosensor 100 is the affixing surface.

The circuit part 130 and the substrate 135 are mounted on the top surface of the base material layer 120. Also, the probe 140 is provided in a form of being embedded in the pressure-sensitive adhesive layer 110 so as to be exposed from a bottom surface 112 of the pressure-sensitive adhesive layer 110. The bottom surface 112 is the affixing surface of the biosensor 100.

The biosensor 100 is assembled by attaching the probes 140 to the base material layer 120 on which the pressure-sensitive adhesive layer 110 is provided, and in a state of the circuit parts 130 and the substrate 135 being arranged on the base material layer 120, covering these components from the top by the cover 170 that is adhered to the base material layer 120. On the substrate 135, the electronic device 150 and the battery 160 are mounted.

The pressure-sensitive adhesive layer 110 is a flat plate-shaped adhesive layer. The pressure-sensitive adhesive layer 110 is oriented to have its longitudinal direction extend in the X axis direction and its short direction extend in the Y axis direction. The pressure-sensitive adhesive layer 110 is supported by the base material layer 120, and is affixed to a bottom surface 121 of the base material layer 120 on the -Z direction side.

As illustrated in FIG. 2, the pressure-sensitive adhesive layer 110 has a top surface 111 and the bottom surface 112. The top surface 111 and the bottom surface 112 are flat surfaces. The pressure-sensitive adhesive layer 110 is a layer with which the biosensor 100 contacts the living body. The bottom surface 112 has pressure-sensitive adhesiveness, and hence, can be affixed to the skin 10 of the living body. The bottom surface 112 is the bottom surface of the biosensor 100, and can be affixed to a biological surface such as the skin 10.

Also, the pressure-sensitive adhesive layer 110 includes through holes 113. Each through hole 113 has the same size and the same position in plan view as a through hole 123 of the base material layer 120, and communicates with the through hole 123.

The material of the pressure-sensitive adhesive layer 110 is not limited in particular as long as being a material having pressure-sensitive adhesiveness, and a material having biocompatibility or the like may be enumerated. As the material of the pressure-sensitive adhesive layer 110, an acryl-based pressure-sensitive adhesive, silicone-based pressure-sensitive adhesive, or the like may be enumerated. Favorably, an acryl-based pressure-sensitive adhesive may be recommended.

The acryl-based pressure-sensitive adhesive contains an acrylic polymer as the main component.

The acrylic polymer is a pressure-sensitive adhesive component. As the acrylic polymer, a polymer polymerized with a monomer component that contains (meth)acrylic ester such as isononyl acrylate, methoxyethyl acrylate, or the like as the main component, and contains a monomer copolymerizable with (meth)acrylic ester such as acrylic acid or the like as an optional component, can be used. The content of the main component among the monomer components is 70 mass% to 99 mass%, and the content of the optional component among the monomer components is 1 mass% to 30 mass%. As the acrylic polymer, for example, a (meth)acrylic esterbased polymer described in Japanese Laid-Open Patent Application No. 2003-342541, or the like can be used.

Favorably, the acryl-based pressure-sensitive adhesive further contains carboxylic acid ester.

The carboxylic acid ester contained in the acryl-based pressure-sensitive adhesive is a pressure-sensitive adhesiveness modifier that reduces the pressure-sensitive adhesiveness of the acrylic polymer, to modify the pressure-sensitive adhesiveness of the pressure-sensitive adhesive layer 110. The carboxylic acid ester is a carboxylic acid ester compatible with an acrylic polymer.

Specifically, the carboxylic acid ester is tri-fatty acid glyceryl, as an example.

The content ratio of carboxylic acid ester is, with respect to 100 parts by mass of the acrylic polymer, favorably 30 parts by mass to 100 parts by mass, and more favorably 50 parts by mass to 70 parts by mass.

The acryl-based pressure-sensitive adhesive may contain a crosslinking agent if necessary. The crosslinking agent is a crosslinking component that crosslinks the acrylic polymer. As the crosslinking agent, a polyisocyanate compound, epoxy compound, melamine compound, peroxide compound, urea compound, metal alkoxide compound, metal chelate compound, metal salt compound, carbodiimide compound, oxazoline compound, aziridine compound, amine compound, or the like may be enumerated. Any of these crosslinking agents may be used alone, or two or more may be used in combination. As the crosslinking agent, favorably, a polyisocyanate compound (polyfunctional isocyanate compound) may be recommended.

The content of the crosslinking agent is, with respect to 100 parts by mass of the acrylic polymer, for example, favorably 0.001 parts by mass to 10 parts by mass, and more favorably 0.01 parts by mass to 1 part by mass.

It is favorable that the pressure-sensitive adhesive layer 110 has an excellent biocompatibility. For example, when the pressure-sensitive adhesive layer 110 undergoes a keratin peeling test, the ratio of keratin-peeled area is favorably 0% to 50%, and more favorably 1% to 15%. As long as the ratio of keratin-peeled area is within a range of 0% to 50%, the load imposed on the skin 10 (see FIG. 2) can be suppressed even if the pressure-sensitive adhesive layer 110 is affixed to the skin 10 (see FIG. 2). Note that the keratin peeling test is measured by a method described in Japanese Laid-Open Patent Application No. 2004-83425.

The moisture permeability of the pressure-sensitive adhesive layer 110 is favorably greater than or equal to 300 g/m²/day, more favorably greater than or equal to 600 g/m²/day, and even more favorably greater than or equal to 1,000 g/m²/day. As long as the moisture permeability of the pressure-sensitive adhesive layer 110 is greater than or equal to 300 g/m²/day, the load imposed on the skin 10 (see FIG. 2) can be suppressed even if the pressure-sensitive adhesive layer 110 is affixed to the skin 10 (see FIG. 2) of the living body.

The pressure-sensitive adhesive layer 110 comes to have biocompatibility by satisfying at least one of the following requirements: the ratio of keratin-peeled area in the keratin peeling test is less than or equal to 50%; and the moisture permeability is greater than or equal to 300 g/m²/day. It is more favorable that the material of the pressure-sensitive adhesive layer 110 satisfies both of the requirements described above. This enables the pressure-sensitive adhesive layer 110 to have a higher biocompatibility more stably.

The thickness between the top surface 111 and the bottom surface 112 of the pressure-sensitive adhesive layer 110 is favorably 10 µm to 300 µm. If the thickness of the pressure-sensitive adhesive layer 110 is within a range of 10 µm to 300 µm, the biosensor 100 can be made thinner, especially in a region other than the electronic device 150 and the battery 160 in the biosensor 100.

The base material layer 120 is a support layer that supports the pressure-sensitive adhesive layer 110, and the pressure-sensitive adhesive layer 110 is bonded to the bottom surface 121 of the base material layer 120. The circuit part 130 and the substrate 135 are arranged on the top surface of the base material layer 120.

The base material layer 120 is a flat plate-shaped (sheet-like) member made of an insulator. The shape of the base material layer 120 in plan view is the same as the shape of the pressure-sensitive adhesive layer 110 in plan view, and these are stacked at aligned positions in plan view.

The base material layer 120 has the bottom surface 121 and a top surface 122. The bottom surface 121 and the top surface 122 are flat surfaces. The bottom surface 121 contacts the top surface 111 of the pressure-sensitive adhesive layer 110 (by pressure-sensitive bonding). The base material layer 120 simply needs to be made of a flexible resin having moderate elasticity, flexibility, and toughness, and may be made of thermoplastic resin such as, for example, polyurethane-based resin, silicone-based resin, acryl-based resin, polystyrene-based resin, vinyl chloride-based resin, and polyester-based resin. The thickness of the base material layer 120 is favorably within a range of 1 µm to 300 µm, more favorably within a range of 5 µm to 100 µm, and even more favorably within a range of 10 µm to 50 µm.

The base material layer 120 has tackiness. The tackiness of the base material layer 120 is represented by a predetermined lower limit value and a predetermined upper limit value.

The predetermined lower limit value simply needs to be a value representing tackiness that is sufficient to allow the circuit part 130 and the substrate 135 to be fixed to the top surface of the base material layer 120 in a state of the base material layer 120 not being stretched. This is primarily because, when assembling the biosensor 100, the circuit part 130 and the substrate 135 need to be fixed on the base material layer 120 when the cover 170 is going to be adhered onto the base material layer 120 in a state of having the circuit part 130 and the substrate 135 arranged. Also, this is also because, after the assembly, the circuit part 130 and the substrate 135 need to be fixed on the base material layer 120 in a state of the base material layer 120 not being stretched. The circuit part 130 and the substrate 135 are arranged on the base material layer 120 without using an adhesive or the like, and in the case described above, are fixed relying on only the tackiness of the base material layer 120.

Also, the predetermined upper limit value simply needs to be a value representing tackiness that is sufficient to allow the circuit part 130 to move so as to be separated from the base material layer 120 in a state of the base material layer 120 being stretched to a certain extent. The circuit part 130 includes a wire 131, a frame 132, and a substrate 133; details of the circuit part 130 will be described later. The wire 131 and the frame 132 are provided on the top surface of the substrate 133, and are formed as a metal plating layer. The frame 132 is provided under the probe 140, and is a rectangular loop-shaped plating layer that surrounds the perimeter of a pressure-sensitive adhesive layer 110A in plan view; and the wire 131 connects the frame 132 to the electronic device 150 and the battery 160.

When the base material layer 120 is stretched, a stretching stress is also applied to the substrate 133. At this time, although the frame 132 hardly deforms as it surrounds the pressure-sensitive adhesive layer 110A, the wire 131 may be stretched. In such a case, in a state of the base material layer 120 being stretched to a certain extent, if the substrate 133 can move to be separated from the base material layer 120, and shift its position with respect to the base material layer 120, breaking of the wire 131 can be suppressed.

From this point of view, the predetermined lower limit value and the predetermined upper limit value are specified for the tackiness of the base material layer 120. The predetermined lower limit value and the predetermined upper limit value will be described later.

The circuit part 130 includes the wire 131, the frame 132, and the substrate 133. More specifically, the circuit part 130 is connected to the electrode via the frame 132, and connected to the electronic device 150 via the wire 131. The biosensor 100 includes two instances of the circuit part 130 as such. The wire 131 and the frame 132 are provided on the top surface of the substrate 133, and formed integrally. The wire 131 connects the frame 132 to the electronic device 150 and the battery 160.

The wire 131 and the frame 132 can be made of copper, nickel, gold, an alloy of these, or the like. The thickness of the wire 131 and the frame 132 is favorably within a range of 0.1 µm to 100 µm, more favorably within a range of 1 µm to 50 µm, and even more favorably within a range of 5 µm to 30 µm.

Each of the two instances of the circuit part 130 is provided corresponding to the two through holes 113 and 123 of the pressure-sensitive adhesive layer 110 and of the base material layer 120, respectively. The wire 131 is connected to the electronic device 150 and a terminal 135A for the battery 160 via wires of the substrate 135. The frame 132 is a rectangular loop-shaped conductive member larger than the opening of the through hole 123 of the base material layer 120.

The substrate 133 has a shape substantially the same as that of the wire 131 and the frame 132 in plan view. Part of the substrate 133 on which the frame 132 is provided has a rectangular loop shape larger than the opening of the through hole 123 of the base material layer 120. The frame 132 and the rectangular loop-shaped part of the substrate 133 on which the frame 132 is provided, are provided to surround the through hole 123 on the top surface of the base material layer 120. The substrate 133 simply needs to be made of an insulator material, and for example, a substrate or film made of polyimide or the like can be used. The base material layer 120 has tackiness; therefore, the substrate 133 is fixed to the top surface of the base material layer 120.

The substrate 135 is a substrate made of an insulator material, to have the electronic device 150 and the battery 160 mounted, and provided on the top surface 122 of the base material layer 120. The substrate 135 is fixed by the tackiness of the base material layer. As the substrate 135, a substrate or film made of polyimide or the like can be used, as an example. On the top surface of the substrate 135, wires and the terminal 135A for the battery 160 are provided. The wires of the substrate 135 are connected to the electronic device 150 and the terminal 135A, and to the wire 131 of the circuit part 130.

The probe 140 is an electrode that comes into contact with the test subject, specifically, an electrode that comes into contact with the skin 10, to detect a biological signal when the pressure-sensitive adhesive layer 110 is affixed to the skin 10. The biological signal is, for example, an electrical signal representing an electrocardiographic waveform, electroencephalogram, pulse, or the like.

The electrode to be used as the probe 140 is produced using a conductive composition that contains at least a conductive polymer and a binder resin as will be described later. Also, the electrode is produced by punching a sheet-like member obtained using the conductive composition, with a mold or the like, to be used as the probe.

The probe 140 has a rectangular shape in plan view, and has holes 140A arranged in a matrix that is larger than the respective through holes 113 and 123 of the pressure-sensitive adhesive layer 110 and of the base material layer 120. At the ends (end parts of four sides) in the X direction and the Y direction of the probe 140, the ladder-like sides of the probe 140 may protrude. The electrode to be used as the probe 140 may have a predetermined patterned shape. As the predetermined electrode patterned shape, a mesh pattern, a stripe pattern, a patterned shape in which the electrode is exposed from multiple locations from the affixing surface.

The fixing tape 145 is an example of a joining part in the present embodiment. The fixing tape 145 is, as an example, a rectangular loop-shaped copper tape. The fixing tape 145 has its bottom surface coated with an adhesive. The fixing tape 145 is provided on the frame 132 so as to surround the four sides of the probe 140 on the outside of the opening of the through holes 113 and 123 in plan view, to fix the probe 140 to the frame 132. The fixing tape 145 may be a tape of metal other than copper.

The fixing tape 145 may be a nonconductive tape such as a resin tape made of a nonconductive resin substrate and an adhesive, other than a tape having a metal layer such as the copper tape. A conductive tape such as a metal tape can join (fix) the probe 140 to the frame 132 of the circuit part 130, and can electrically connect these parts, and hence, is favorable.

The probe 140 is fixed to the frame 132 by the rectangular loop-shaped fixing tape 145 that covers edge parts along the four sides, in a state of the edge parts along the four sides being arranged on the frame 132. The fixing tape 145 is adhered to the frame 132 through gaps such as the holes 140A in the probe 140.

In a state of the probe 140 having its edge parts along the four sides fixed on the frame 132 by the fixing tape 145 in this way, the pressure-sensitive adhesive layer 110A and the base material layer 120A are overlaid on the fixing tape 145 and the probe 140; and when the pressure-sensitive adhesive layer 110A and the base material layer 120A are pressed downward, the probe 140 is pushed along the inner walls of the through holes 113 and 123, and the pressure-sensitive adhesive layer 110A is pushed into the interior of the holes 140A in the probe 140.

The probe 140 is pushed down to a position at which its center part becomes substantially flush with the bottom surface 112 of the pressure-sensitive adhesive layer 110, in a state of its edge parts along the four sides being fixed to the frame 132 by the fixing tape 145. Therefore, if having the probe 140 come into contact with the skin 10 of the living body (see FIG. 2), the pressure-sensitive adhesive layer 110A can be adhered to the skin 10, and the probe 140 can be firmly adhered to the skin 10.

It is favorable that the thickness of the probe 140 is thinner than the thickness of the pressure-sensitive adhesive layer 110. The thickness of the probe 140 is favorably within a range of 0.1 µm to 100 µm, and more favorably within a range of 1 µm to 50 µm.

Also, the surrounding part (rectangular loop-shaped part) surrounding the central part of the pressure-sensitive adhesive layer 110A in plan view is positioned above the fixing tape 145. In FIG. 2, although the top surface of the pressure-sensitive adhesive layer 110A is generally flat, the center part may be recessed downward compared to the surrounding part. The base material layer 120A is overlaid on the generally flat top surface of the pressure-sensitive adhesive layer 110A.

The pressure-sensitive adhesive layer 110A and the base material layer 120A as such may be made of the same materials as the pressure-sensitive adhesive layer 110 and the base material layer 120, respectively. Also, the pressure-sensitive adhesive layer 110A may be made of a material different from that of the pressure-sensitive adhesive layer 110. Also, the base material layer 120A may be made of a material different from that of the base material layer 120.

Note that in FIG. 2, although the thicknesses of the respective parts are exaggerated, in practice, the thicknesses of the pressure-sensitive adhesive layer 110 and 110A is within a range of 10 µm to 300 µm, and the thicknesses of the base material layer 120 and 120A is within a range of 1 µm to 300 µm. Also, the thicknesses of the wire 131 is within a range of 0.1 µm to 100 µm, the thicknesses of the substrate 133 is around several 100 µm, and the thicknesses of the fixing tape 145 is within a range of 10 µm to 300 µm.

Also, as illustrated in FIG. 2, in the case where the probe 140 directly contacts the frame 132, and the electrical connection is secured, the fixing tape 145 may be a tape made of resin or the like that does not have electrical conductivity.

Also, in FIG. 2, the fixing tape 145 covers the side surfaces of the frame 132 and the substrate 133 in addition to the probe 140, and reaches the top surface of the base material layer 120. However, the fixing tape 145 simply needs to have the probe 140 and the frame 132 joined, and hence, does not need to reach the top surface of the base material layer 120; does not need cover the side surfaces of the substrate 133; and does not need cover the side surfaces of the frame 132.

Also, the substrate 133 and the two substrates 135 may be one integrated substrate. In this case, the wires 131, the two frames 132, and the terminal 135A are provided on a surface of the one substrate, to have the electronic device 150 and the battery 160 mounted.

It is favorable to produce the electrode to be used as the probe 140, by heat curing and molding a conductive composition as follows. The conductive composition contains a conductive polymer, a binder resin, and at least one of a crosslinking agent and a plasticizing agent.

As the conductive polymer, for example, polythiophene, polyacetylene, polypyrrole, polyaniline, polyphenylene vinylene, or the like can be used. Any of these polymers may be used alone, or two or more may be used in combination. Among these polymers, it is favorable to use a polythiophene compound. From the viewpoints of having a lower contact impedance with a living body and high electrical conductivity, it is more favorable to use PEDOT/PSS doped with polystyrenesulfonic acid (poly4-styrenesulfonate; PSS) in poly3,4-ethylenedioxythiophene (PEDOT).

The content of the conductive polymer is, with respect to 100 parts by mass of the conductive composition, favorably 0.20 to 20 parts by mass. As long as the content is within the range described above, excellent electrical conductivity, toughness, and flexibility can be imparted to the conductive composition. The content of the conductive polymer is, with respect to the conductive composition, more favorably 2.5 to 15 parts by mass, and even more favorably 3.0 to 12 parts by mass.

As the binder resin, a water-soluble polymer, a water-insoluble polymer, or the like can be used. As the binder resin, it is favorable to use a water-soluble polymer from the viewpoint of compatibility with other components contained in the conductive composition. Note that the water-soluble polymer includes a hydrophilic polymer that is hydrophilic though not completely soluble in water.

As the water-soluble polymer, a hydroxyl group-containing polymer or the like can be used. As the hydroxyl group-containing polymer, sugars such as agarose or the like, polyvinyl alcohol (PVA), modified polyvinyl alcohol, a copolymer of acrylic acid and sodium acrylate, or the like can be used. Any of these polymers may be used alone, or two or more may be used in combination. Among these polymers, polyvinyl alcohol or modified polyvinyl alcohol is favorable, and modified polyvinyl alcohol is more favorable.

As the modified polyvinyl alcohol, acetoacetyl group-containing polyvinyl alcohol, diacetone acrylamide modified polyvinyl alcohol, or the like may be enumerated. Note that as the diacetone acrylamide modified polyvinyl alcohol, for example, a diacetone acrylamide modified polyvinyl alcohol-based resin (DA modified PVA-based resin) described in Japanese Laid-Open Patent Application No. 2016-166436 can be used.

The content of the binder resin is, with respect to 100 parts by mass of the conductive composition, favorably 5 to 140 parts by mass. As long as the content is within the range described above, excellent electrical conductivity, toughness, and flexibility can be imparted to the conductive composition. The content of the binder resin is, with respect to the conductive composition, more favorably 10 to 100 parts by mass, and even more favorably 20 to 70 parts by mass.

A crosslinking agent and a plasticizing agent have functions of giving toughness and flexibility to the conductive composition. By imparting the flexibility to the formed object of the conductive composition, an electrode having elasticity is obtained. By using this electrode, the probe 140 having elasticity can be produced.

Note that the toughness is a property that makes excellent strength and elongation compatible with each other. The toughness does not include a property in which either one of the strength or elongation is remarkably excellent whereas the other is remarkably inferior, but includes a property in which both are balanced.

The flexibility is a property that, when a formed object (electrode sheet) of the conductive composition is bent, damage such as fracture in the bent part can be prevented.

The crosslinking agent crosslinks the binder resin. By having the crosslinking agent contained in the binder resin, the toughness of the conductive composition can be improved. It is favorable that the crosslinking agent has reactivity with a hydroxyl group. If the crosslinking agent has reactivity with a hydroxyl group, in the case where the binder resin is a hydroxyl group-containing polymer, the crosslinking agent can react with hydroxyl groups of a hydroxyl group-containing polymer.

As the crosslinking agent, a zirconium compound such as zirconium salt; a titanium compound such as titanium salt; a borate such as boric acid; an isocyanate compound such as blocked isocyanate; an aldehyde compound such as dialdehyde such as glyoxal; an alkoxyl group-containing compound, a methylol group-containing compound, or the like may be enumerated. Any of these agents may be used alone, or two or more may be used in combination. Among these agents, a zirconium compound, isocyanate compound, or aldehyde compound is favorable from the viewpoint of the reactivity and the safety.

The content of the crosslinking agent is, with respect to 100 parts by mass of the conductive composition, favorably 0.2 to 80 parts by mass. As long as the content is within the range described above, excellent toughness and flexibility can be imparted to the conductive composition. The content of the crosslinking agent is more favorably 1 to 40 parts by mass, and even more favorably 3.0 to 20 parts by mass.

The plasticizing agent improves the tensile elongation and the flexibility of the conductive composition. As the plasticizing agent, glycerin, ethylene glycol, propylene glycol, sorbitol, a polyol compound of these polymers or the like, N-methylpyrrolidone (NMP), an aprotonic compound such as dimethyl formaldehyde (DMF), N-N'-dimethylacetamide (DMAC), dimethyl sulfoxide (DMSO), or the like may be enumerated. Any of these agents may be used alone, or two or more may be used in combination. Among these agents, glycerin is favorable from the viewpoint of compatibility with the other components.

The content of the plasticizing agent is, with respect to 100 parts by mass of the conductive composition, favorably 0.2 parts by mass to 150 parts by mass. As long as the content is within the range described above, excellent toughness and flexibility can be imparted to the conductive composition. The content of the plasticizing agent is, with respect to 100 parts by mass of the conductive composition, more favorably 1.0 parts by mass to 90 parts by mass, and even more favorably 10 parts by mass to 70 parts by mass.

As for the crosslinking agent and the plasticizing agent, at least one of these may be contained in the conductive composition. By having at least one of the crosslinking agent and the plasticizing agent contained in the conductive composition, the formed object of the conductive composition can improve the toughness and the flexibility.

In the case where the conductive composition contains the crosslinking agent but does not contain the plasticizing agent, the formed object of the conductive composition can improve the toughness, namely, both the tensile strength and the tensile elongation, and simultaneously, can improve the flexibility.

In the case where the plasticizing agent is contained in the conductive composition, but the crosslinking agent is not contained, the formed object of the conductive composition can be improved in terms of the tensile elongation, and hence, the toughness of the formed object of the conductive composition can be improved as a whole. Also, the flexibility of the formed object of the conductive composition can be improved.

It is favorable that both the crosslinking agent and the plasticizing agent are contained in the conductive composition. By having both the crosslinking agent and the plasticizing agent contained in the conductive composition, more outstanding toughness can be imparted to the formed object of the conductive composition.

In addition to the above components, the conductive composition can optionally contain a variety of well-known additives such as a surfactant, a softening agent, a stabilizer, a leveling agent, an antioxidant, an anti-hydrolysis agent, a swelling agent, a thickener, a colorant, a bulking agent, and the like, by appropriate ratios, if necessary. As the surfactant, a silicone-based surfactant and the like may be enumerated.

The conductive composition is prepared by mixing the components described above by the ratios described above.

The conductive composition may optionally contain a solvent by an appropriate ratio, if necessary. In this way, an aqueous solution of the conductive composition (the aqueous solution of the conductive composition) is prepared.

As the solvent, an organic solvent or an aqueous solvent can be used. As the organic solvent, for example, ketones such as acetone, methyl ethyl ketone (MEK), or the like; ester such as ethyl acetate; ethers such as propylene glycol monomethyl ether or the like; amides such as N,N-dimethylformamide, or the like, may be enumerated. As the aqueous solvent, for example, water; alcohol such as methanol, ethanol, propanol, isopropanol, or the like, may be enumerated. Among these solvents, it is favorable to use an aqueous solvent.

At least one of the conductive polymer, the binder resin, and the crosslinking agent may be used as an aqueous solution dissolved in a solvent. In this case, as the solvent, an aqueous solvent described above is favorable.

The electronic device 150 is mounted on the top surface 122 of the base material layer 120, and electrically connected to the wires 131. The electronic device 150 processes biological signals obtained via the electrodes used as the probes 140. The electronic device 150 has a rectangular shape in cross sectional view. The bottom surface (in the -Z direction) of the electronic device 150 is provided with terminals. As the material of the terminals of the electronic device 150, solder, conductive paste, or the like may be enumerated.

As illustrated in FIG. 1, the electronic device 150 includes, as an example, an application specific integrated circuit (ASIC) 150A, a micro processing unit (MPU) 150B, a memory 150C, and a wireless communication unit 150D; and is connected to the probes 140 via the circuit parts 130 and the battery 160.

The ASIC 150A includes an A/D (Analog to Digital) converter. The electronic device 150 is driven by electric power supplied from the battery 160, to obtain biological signals measured by the probes 140. The electronic device 150 executes processing such as filtering and digital conversion on the biological signals, and the MPU 150B calculates an arithmetic mean of values of the biological signals obtained multiple times, to store the mean in the memory 150C. The electronic device 150 can obtain biological signals continuously, as an example, for 24 hours or longer. In some cases, the electronic device 150 measures biological signals for a long period of time; therefore, various ideas are incorporated to reduce the electric power consumption.

The wireless communication unit 150D is a transceiver used when a test device of an evaluation test reads biological signals stored in the memory 150C during the evaluation test via the wireless communication, and executes communication, as an example, at 2.4 GHz. The evaluation test is a test, as an example, compliant with the standard of JIS 60601-2-47. The evaluation test is a test executed after completion of a biosensor, to verify operations of the biosensor to detect biological signals as a medical device. The evaluation test requires an attenuation factor of a biological signal extracted from the biosensor being less than 5% with respect to a biological signal input into the biosensor. This evaluation test is to be executed for all completed products.

As illustrated in FIG. 2, the battery 160 is provided on the top surface 122 of the base material layer 120. As the battery 160, a lead battery, a lithium ion secondary battery, or the like can be used. The battery 160 may be a button battery. The battery 160 is an example of a battery. The battery 160 has two terminals (not illustrated) provided on its bottom surface. The two terminals of the battery 160 are electrically connected to two terminals 131B, respectively. The capacity of the battery 160 is set so that the electronic device 150 can measure biological signals, as an example, for 24 hours or longer.

The cover 170 covers the base material layer 120, the circuit parts 130, the substrate 135, the probes 140, the fixing tapes 145, the electronic device 150, and the battery 160. The cover 170 has a base part 170A and a protruding part 170B protruding in the +Z direction from the center of the base part 170A. The base part 170A is a part positioned at the periphery of the cover 170 in plan view, and is a part positioned lower than protruding part 170B. A recessed part 170C is provided below the protruding part 170B. In the cover 170, the bottom surface of the base part 170A is adhered to the top surface 122 of the base material layer 120. In the recessed part 170C, the substrate 135, the electronic device 150, and the battery 160 are housed. The cover 170 is bonded to the top surface 122 of the base material layer 120 in a state of having the electronic device 150, the battery 160, and the like housed in the recessed part 170C.

In addition to the role of serving as a cover for protecting the circuit parts 130, the electronic device 150, and the battery 160 on the base material layer 120, the cover 170 has a role of serving as a shock absorbing layer to protect the interior components from shocks applied to the biosensor 100 from the top surface side. As the cover 170, for example, silicone rubber, soft resin, urethane, or the like can be used.

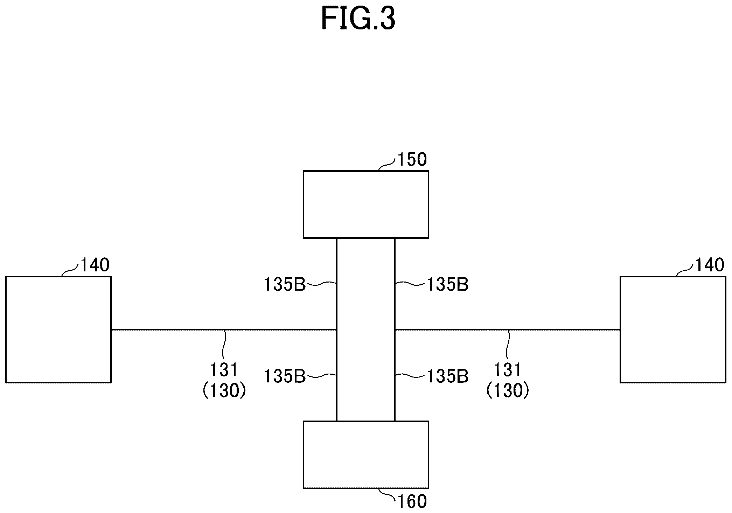

FIG. 3 is a diagram illustrating a circuit configuration of the biosensor 100. Each of the probes 140 is connected to the electronic device 150 and the battery 160 via the wire 131 and the wire 135B of the substrate 135. The two probes 140 are connected in parallel to the electronic device 150 and the battery 160.

Next, the predetermined lower limit value and the predetermined upper limit value of the tackiness of the base material layer 120 will be described. As a method of examining the degree of "stickiness" as the most characteristic property of adhesives, a ball rolling method called J. Dow ball tack is adopted in the JIS standards. Steel balls of varying sizes are rolled on the surface of an adhesive set as a tilted surface (30 degrees), to evaluate the "stickiness" whether the balls stop or fall down. Stopping of a larger steel ball means a stronger tackiness (i.e., a value indicating the tackiness is larger).

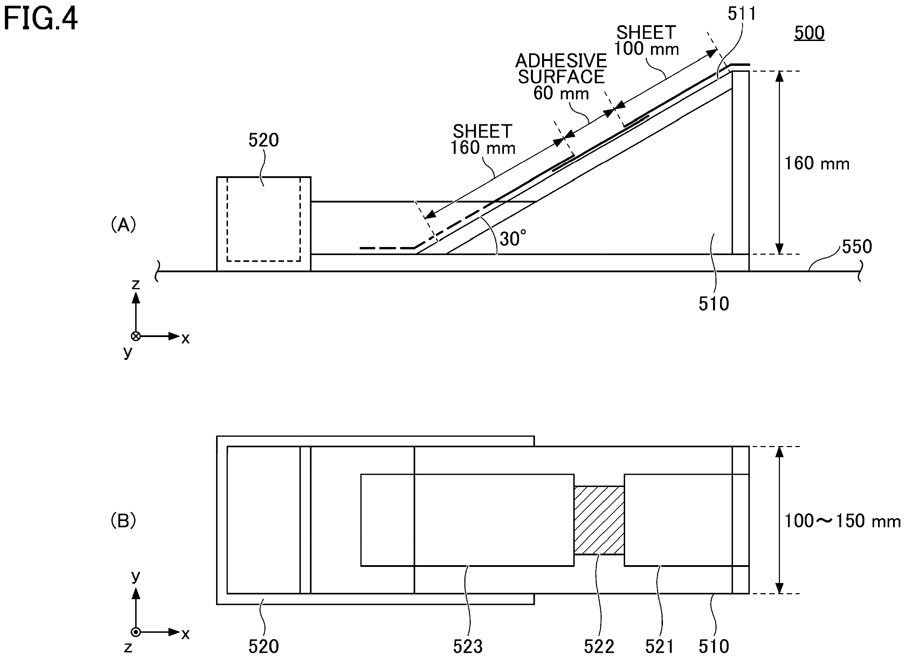

FIG. 4 is a diagram illustrating a ball rolling device 500 used in the J. Dow ball tack as a ball rolling method in the JIS standards. The ball rolling device 500 includes a ball rolling table 510 having a slope 511 and a ball receiver 520. The slope 511 is has an angle of 30 degrees (tilt angle) with respect to the horizontal plane. An orthogonal xyz coordinate system denoted by lowercase letters of xyz is used here for the description.

A sheet 521, an adhesive layer 522, and a sheet 523 are affixed to the slope 511 from the top to the bottom. Along the slope 511, the interval of the sheet 521 is 100 mm long, the interval of the adhesive layer 522 is 60 mm long, and the interval of the sheet 523 is 150 mm long. The ball receiver 520 is provided ahead of the interval of the sheet 523. The sheet 521 serves as an approach when rolling a steel ball on the slope 511, and the sheet 523 serves as a runway in the case where the steel ball does not stop at the adhesive layer 522, and rolls away.

The ball rolling device 500 is horizontally fixed to a measuring table 550 by using a level. The adhesive layer 522 used on the slope 511 simply needs to be a sample having a size of a width of 10 mm in the y-axis direction and a length of 70 mm or longer along the slope 511. The adhesive layer 522 is fixed to a predetermined position on the slope 511 with the adhesive surface oriented upward, and the sheet 521 serving as the approach is affixed to the upper end of the adhesive layer 522.

The length of the sheet 521 serving as the approach is set to 100 mm. When fixing the sheet 521 and the adhesive layer 522 to the slope 511, care should be taken so that these are not floated, wrinkled, or bent. In the case where an edge of the sheet 521 or the adhesive layer 522 is curved and floated over the slope 511, that part is fixed to the slope 511 by another adhesive tape or the like. Then, the adhesive surface is left in the center of the slope 511 in the width direction (y direction) by a length of 50 mm to 100 mm, and the lower end is covered with the sheet 523 appropriately.

In this state, multiple types of steel balls having diameters different from one another are rolled down from the top end of the slope 511, and a maximum diameter (mm) from among the diameters of the steel balls stopped within the range of the adhesive layer 522 is taken as the measured value of the test.

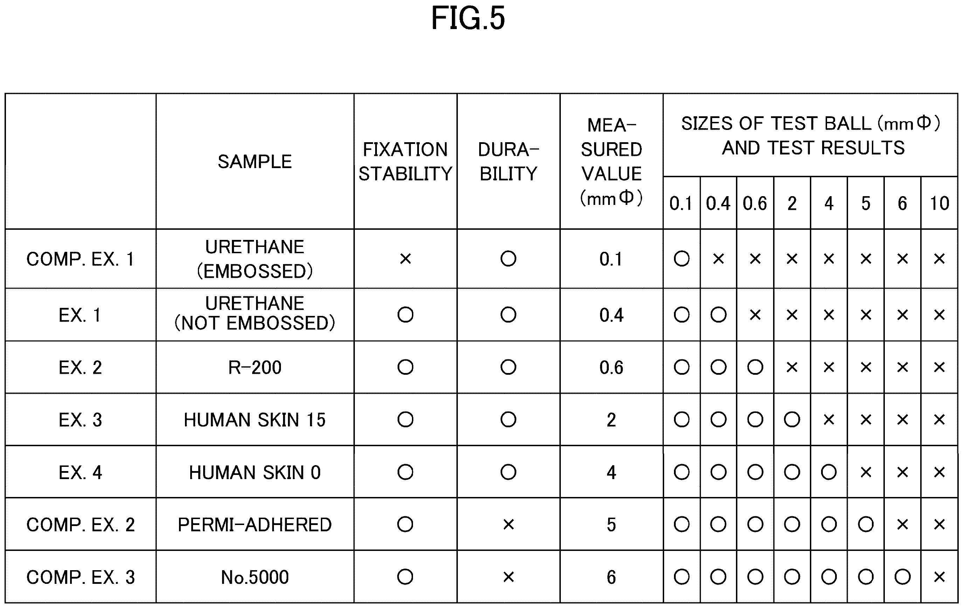

FIG. 5 is a table showing test results. FIG. 5 shows results of tests using seven samples (Examples 1 to 4 and Comparative examples 1 to 3) as the adhesive layer 522. Here, Examples 1 to 4 are samples that passed in terms of both the fixation stability and the durability (circle marks), whereas Comparative examples 1-3 are samples that failed in at least one of the fixation stability and the durability (cross marks).

The fixation stability represents strength of the tackiness of the base material layer 120, and was evaluated as passing (circle marks) if the circuit part 130 did not fall off in the case where the circuit part 130 in a state of being placed on the base material layer 120 was inverted upside down by 180 degrees, or was evaluated as failing (cross marks) if the circuit part 130 fell off or was partially peeled off.

Also, the durability was evaluated as passing (circle marks) if the wire 131 of the circuit part 130 did not break, or was evaluated as failing (cross marks) if the wire 131 of the circuit part 130 broke, when the pressure-sensitive adhesive layer 110 as the sample is affixed to an 8-µm thick urethane substrate as the base material layer 120 and only the urethane substrate was stretched in a state of the circuit part 130 being placed on the base material layer 120.

The following materials were used in Examples 1 to 4: urethane (not embossed); a film for surface protection named R-200 (a polyester-based film) manufactured by Nitto Denko Corporation; a human skin-imitating gel made of ultra-soft urethane (hardness 15) manufactured by Exseal Co., Ltd.; and the same (hardness 0).

Also, the following materials were used in Comparative examples 1 to 3, respectively: urethane (embossed); a product called Permiroll in Japan (adhesive layer) manufactured by Nitto Denko Corporation; and a double-sided tape numbered as No. 5000NS manufactured by Nitto Denko Corporation.

Results of tests in which steel balls having the respective diameters of 0.1 (mm ϕ), 0.4 (mm ϕ), 0.6 (mm ϕ), 2 (mm ϕ), 4 (mm ϕ), 5 (mm ϕ), 6 (mm ϕ), and 10 (mm ϕ) were rolled down from the top end of the slope 511 are shown with circle marks and cross marks, where a circle mark corresponds to a case where the ball stopped on the adhesive layer 522, and a cross mark corresponds to a case where the ball did not stop on the adhesive layer 522 and rolled down onto the sheet 523. The measured value of each row in FIG. 5 indicates a maximum diameter among test results marked with circle marks.

As shown in FIG. 5, the measured values of Examples 1 to 4 are 0.4 (mm ϕ), 0.6 (mm ϕ), 2 (mm ϕ), and 4 (mm ϕ), respectively, and the measured values in Comparative examples 1 to 3 were 0.1 (mm ϕ), 5 (mm ϕ), and 6 (mm ϕ), respectively.

Also, as for the fixation stability, except for Comparative example 1 marked with a cross mark, all the other were marked with circle marks. As for the durability, Examples 1 to 4 were marked with circle marks, and Comparative examples 1 to 3 were marked with cross marks.

From the above, it was understood that the tackiness of the base material layer 120 could be represented by a maximum diameter from among the diameters of the steel balls that stopped in the ball rolling method of the J. Dow ball tack, is greater than or equal to 0.4 mm and less than or equal to 4 mm.

As described above, in the biosensor 100 according to the embodiment, the lower limit value of the tackiness of the base material layer 120 on which the circuit part 130 is mounted, is a value that allows the circuit part 130 and the substrate 135 to be fixed to the top surface of the base material layer 120 in a state of the base material layer 120 not being stretched. As such a lower limit value, a maximum diameter from among the diameters of the steel balls that stop in the ball rolling method of the J. Dow ball tack that is 0.4 mm ϕ, may be adopted.

Using such a lower limit value, in a state of the circuit part 130 being placed on the base material layer 120 while producing the biosensor 100, the circuit part 130 can be fixed to the base material layer 120, and positional misalignment would not occur.

Also, the upper limit value of the base material layer 120 is a value that allows the circuit part 130 to relatively move so as to be separated from the base material layer 120 in a state of the base material layer 120 being stretched to a certain extent. Therefore, when a living body moves in a state of having the biosensor 100 affixed, if the lower part of the substrate 133 in the base material layer 120 is stretched, the substrate 133 is separated from the base material layer 120; therefore, the stress applied to the base material layer 120 can be suppressed from being applied to the wire 131. As such an upper limit value, a maximum diameter from among the diameters of the steel balls that stop in the ball rolling method of the J. Dow ball tack that is 4 mm ϕ, may be adopted.

By using such an upper limit value, even if the lower part of the substrate 133 in the base material layer 120 is stretched, breaking of the wire 131 can be suppressed. This effect can be brought not only when the living body moves in a state of having the biosensor 100 affixed, but also when affixing the biosensor 100 to the living body, and when peeling off the biosensor 100 from the living body.

Thus, the electrode joining structure 100A and the biosensor 100, in which breaking of a wire can be suppressed, can be provided.

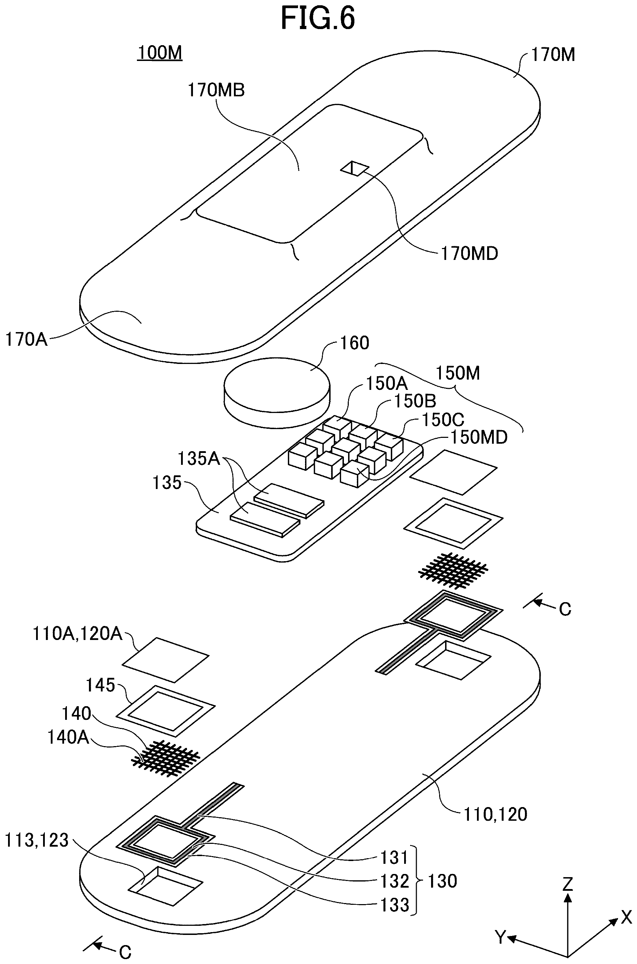

Also, in the configuration described above, the electronic device 150 has the wireless communication unit 150D; however, a configuration as illustrated in FIG. 6 may be adopted. FIG. 6 is an exploded view illustrating a biosensor 100M according to a modified example.

A biosensor 100M includes an electronic device 150M and a cover 170M in place of the electronic device 150 and the cover 170 illustrated in FIG. 1.

The electronic device 150M includes an ASIC 150A, an MPU 150B, a memory 150C, and a connector 150MD4. The electronic device 150M has the connector 150MD4 in place of the wireless communication unit 150D of the electronic device 150 illustrated in FIG. 1.

Also, the cover 170M has a through hole 170MD4 provided directly above the connector 150MD4. The through hole 170MD4 is provided in a projecting part 170MB, into which a connector of a test device for evaluation test is inserted to be connected to the connector 150MD4.

In the biosensor 100M, in an evaluation test, biological signals stored in the memory 150C are read by a test device via the connector 150MD4. Note that the through hole 170MD4 of the cover 170M may be filled with the same material as that of the cover 170M after completion of the evaluation test.

As described above, the electrode joining structure and the biosensor have been described according to the exemplary embodiments of the present invention; note that the present invention is not limited to the embodiments specifically disclosed herein, and various variations and alterations can be made without deviating from the subject matters described in the claims.

This application claims priority under Japanese Patent Application No. 2019-058327 filed with the Japanese Patent Office on March 26, 2019, and the entire contents of which are incorporated herein by reference.

### [Description of Reference Codes]

- 100: biosensor
- 100A: electrode joining structure
- 110: pressure-sensitive adhesive layer
- 120: base material layer
- 130: circuit part
- 140: probe
- 150: electronic device
- 160: battery
- 170: cover

## Claims

1. An electrode joining structure, comprising:
a pressure-sensitive adhesive layer having an affixing surface to be affixed to a test subject;
an electrode containing a conductive polymer having elasticity, and configured to be exposed from the affixing surface of the pressure-sensitive adhesive layer;
a base material layer provided to be overlaid on a surface opposite to the affixing surface of the pressure-sensitive adhesive layer, and having tackiness represented by a maximum diameter from among diameters of steel balls that stop in a ball rolling method called J. Dow ball tack, the maximum diameter being greater than or equal to 0.4 mm and less than or equal to 4 mm;
a substrate provided on the base material layer;
a wire provided on the substrate and connected to the electrode; and
a joining part joining the electrode and the wire.

2. The electrode joining structure as claimed in claim 1, wherein the wire is provided on the substrate.

3. The electrode joining structure as claimed in claim 2, wherein the wire is constituted with a plating layer produced by plating on the substrate.

4. The electrode joining structure as claimed in any one of claims 1 to 3, wherein stiffness of the substrate is higher than stiffness of the base material layer.

5. A biosensor comprising:
the electrode joining structure as claimed in any one of claims 1 to 4; and
an electronic device provided on the base material layer, and configured to process a biological signal obtained via the electrode.
